# EUROPEAN PATENT APPLICATION

(11) **EP 4 265 120 A1**
(43) Date of publication of application: **25.10.2023**
(21) Application number: 20966063.8
(22) Date of filing: 18.12.2020
(51) Int. Cl.: A23J 1/20, A23J 3/08, A23J 7/00, A23C 9/152, A23C 1/16, A23C 7/04, A23L 33/19, A61K 8/98

(54) **WHEY-DERIVED POLAR LIPIDS-CONCENTRATED FRACTION COMPOSITION AND USE THEREOF**

(71) Applicant: Solus Biotech Co., Ltd., Iksan-si, Jeollabuk-do 54588 (KR)
(72) Inventor: LEE, Kwanhyoung, Yongin-si, Gyeonggi-do 16858 (KR); KIM, Ara, Yongin-si, Gyeonggi-do 16858 (KR); LEE, Juyeon, Yongin-si, Gyeonggi-do 16858 (KR); JANG, Hyuncheol, Yongin-si, Gyeonggi-do 16858 (KR); KIM, Youngbeom, Yongin-si, Gyeonggi-do 16858 (KR); SEO, Daebang, Yongin-si, Gyeonggi-do 16858 (KR)
(74) Representative: Office Freylinger
(86) International application number: PCT/KR2020/018698
(87) International publication number: WO 2022/131409

(57) **Abstract**

The present invention relates to a whey-derived polar lipid-concentrated fraction composition, being a single-phase composition derived from a whey protein concentrate, wherein the composition is derived from a fraction obtained by treating the whey protein concentrate with a water-containing spirit, a total lipid content is 90 percent by weight (wt%) or more with respect to a dry weight of the composition, and a phospholipid content is 35 wt% or more with respect to the total lipid weight. According to the present invention, a whey-derived polar lipid-concentrated fraction composition in a stable form that maximizes emulsification and physiological functionality may be prepared.

## Description

### TECHNICAL FIELD

The present invention relates to a whey-derived polar lipids-concentrated fraction composition, a method for preparing the same, and a use of the same.

### BACKGROUND ART

Mammals, including humans, deliver lipid nutrients in the form of membranes of fat milk globules, and complex lipid components such as polar lipids, especially phospholipids and sphingolipids, and cholesterol are known. Various preparing methods and physiological functions are known for these milk fat globular complex lipid components.

In EP1814399, a process for obtaining a concentrated fraction of a complex lipid such as phospholipids, commonly referred to as beta serum, through a phase transition process during the production of butter and butter oil, and its use for infant food have been described. However, the method of using supercritical carbon dioxide suggested in the above literature as a method of obtaining a polar fat concentrate has downsides in that a production cost is high and operating conditions such as subcritical conditions should be implemented by mixing water or ethanol due to the nature of the supercritical fluid having non-polar properties.

Furthermore, in WO2003/071875, ultrafiltration and diafiltration methods have been proposed as methods of concentrating polar lipids such as sphingolipids from whey. However, this method has downsides in that the content of polar lipids that may be concentrated is limited, and protein is necessarily included in excess, so it inevitably has to go through a powder form to use the material, and when processing according to the purpose, because ingredients of a water phase and ingredients of an oil phase are mixed, it is difficult to apply the ingredients of the oil phase when using the water phase, and it is difficult to apply the ingredients of the water phase when using the oil phase.

In addition, WO2008/009636 discloses a method for concentrating polar lipids in the form of phospholipids through ultrafiltration and diafiltration, or ultrafiltration, from pasteurized milk cream, but the polar lipid concentrate through this method inevitably contains large amounts of lactose and casein, and thus when the polar lipids were concentrated to a high content, the polar lipids, non-polar lipids, and aqueous components were inconsistent with each other, causing problems such as layer separation and precipitation.

In addition, WO2009/066685 discloses a method of using milk-derived polar lipids for the purpose of improving the sensation of nerve terminals through oral ingestion or skin application, but specifically a butter-derived polar lipid composition has been disclosed. In addition, Japanese Patent JP2003/070669 discloses that a whey-derived phospholipid composition may be used to prevent rotavirus, but the phospholipid content did not exceed 3%.

### DETAILED DESCRIPTION OF THE INVENTION

### TECHNICAL OBJECTIVES

The present invention is directed to solving disadvantages that a milk fat membrane lipid concentrate prepared from a byproduct of a conventional butter oil preparing process is difficult to be standardized due to lactose and casein, and inevitably contains a large amount of water-soluble components when concentrating polar lipid components.

Specifically, the present invention is directed to providing a highly concentrated phospholipid composition in a stable form that maximizes emulsification and physiological functionality.

In addition, the present invention is directed to providing a food having a high phospholipid content and easy drinkability compared to a conventional milk lecithin (derived from butter).

### TECHNICAL SOLUTION TO THE PROBLEM

In order to achieve the above objectives, the present invention provides a whey-derived polar lipid-concentrated fraction composition, being a single-phase composition derived from a whey protein concentrate, wherein the composition is derived from a fraction obtained by treating the whey protein concentrate with a water-containing spirit, a total lipid content is 90 percent by weight (wt%) or more with respect to a dry weight of the composition, and a phospholipid content is 35 wt% or more with respect to the total lipid weight.

The composition may further preferably include a reducing sugar in an amount of 1.35 wt% or less with respect to the dry weight of the composition.

The composition may further preferably include 1 to 10 wt% of cholesterol with respect to the dry weight of the composition.

The composition may further preferably include 1 to 10 wt% of protein with respect to the dry weight of the composition.

It may be preferable that 0.5 to 1.5 parts by weight of the phosphatidylethanolamine and 0.5 to 1.5 parts by weight of the sphingomyelin are included with respect to 1 part by weight of the phosphatidylcholine.

Another aspect of the present invention provides a method of preparing a whey-derived polar lipid-concentrated fraction composition, the method including: 1) preparing a whey protein concentrate by concentrating a whey solution; 2) treating the whey protein concentrate with a water-containing spirit and then separating the whey protein concentrate into a spirit-soluble material and a spirit-insoluble material; and 3) obtaining a whey-derived polar lipid-concentrated fraction by removing ethanol and moisture from the spirit-soluble material.

Another aspect of the present invention provides uses of the composition such as for food, for cosmetics, and for pharmaceuticals.

### EFFECTS OF THE INVENTION

According to the present invention, a composition in which a lipid component of a milk fat membrane is stable may be prepared. Specifically, disadvantages of the milk fat membrane polar lipid concentrate prepared from a byproduct of a conventional butter oil preparing process which is difficult to be standardized due to lactose and casein and inevitably contains a large amount of water-soluble components when concentrating a polar lipid components may be addressed, thereby providing a whey-derived polar lipid-concentrated fraction composition in a stable form that maximizes emulsification and physiological functionality.

According to the present invention, a food having a high phospholipid content and easy drinkability compared to a conventional milk lecithin (derived from butter) may be prepared, and in particular, a powdered milk product close to breast milk may be manufactured. In addition, a pharmaceutical product having a use for improving intestinal health may be manufactured.

### BRIEF DESCRIPTION OF THE DRAWINGS

FIG. 1 illustrates a difference between a whey-derived polar lipid-concentrated fraction and a butter-derived polar lipid (BPL)-concentrated fraction. The whey-derived polar lipid-concentrated fraction was in a homogeneous solid state at 5°C and a homogeneous oil-phase liquid state at 60°C, but a phase separation was observed in the butter-derived polar lipid-concentrated fraction.
FIG. 2 illustrates a result of analyzing a sugar content of the whey-derived polar lipid-concentrated fraction of Embodiment 2 by high performance liquid chromatography.
FIG. 3 illustrates a result of analyzing a phospholipid content of the whey-derived polar lipid-concentrated fraction of Embodiment 2 by high performance liquid chromatography.
FIG. 4 illustrates a comparison of water-phase dispersion states of the whey-derived polar lipid-concentrated fraction of Embodiment 2 (left) and a milk neutral lipid (right).
FIG. 5 illustrates a comparison of oil-phase dispersion states of the whey-derived polar lipid-concentrated fraction of Embodiment 2 (left) and the milk neutral lipid (right).
FIG. 6 illustrates an anti-inflammatory effect of the whey-derived polar lipid-concentrated fraction.

### BEST MODES FOR IMPLEMENTATION OF THE INVENTION

Hereinafter, the present invention will be described.

A conventional method for preparing a concentrated phospholipid composition may inevitably result in containing a large amount of non-lipid components such as lactose and casein, and when polar lipids are concentrated to a high content, polar lipids, non-polar lipids, and aqueous components are inconsistent with each other, resulting in layer separation, precipitation, and the like to cause poor flow, and a production cost was also high.

Accordingly, the present inventors prepared a single-phase polar lipid concentrate by applying a spirit extraction process to a whey instead of a butter oil, thereby obtaining a polar lipid concentrated composition capable of preventing layer separation and precipitation and achieving excellent flowability. The present invention is based on this.

The present invention relates to a single-phase composition derived from a fraction obtained by treating a whey protein concentrate with a water-containing spirit.

As used herein, "the single-phase composition" means that "a content of the total lipid is 90 wt% or more with respect to a dry weight of the composition".

As used herein, the term "whey protein concentrate" refers to a high-quality whey protein that is concentrated by removing non-protein components by applying methods such as ion exchange filtering, ultra-filtering, and micro-filtering to a whey.

As used herein, the term "fraction" refers to a form of an extract obtained by immersing an extraction target material in two or more immiscible solvents and extracting the extraction target material into two or more solvent layers.

As used herein, the whey refers to a lactose, a whey protein, minerals, and the like that are separated from casein when producing cheese from milk.

As used herein, the whey may be a sweet whey or an acid whey.

As used herein, the whey protein refers to a liquid byproduct containing protein, lactose, minerals, vitamins, inorganic components, and the like separated during cheese production or casein production.

In the composition of the present invention, a content of phospholipid, which is a polar lipid component, from among components of a milk fat membrane is 35 percent by weight (wt%) or more with respect to the total lipid weight,

In the present invention, the phospholipid as a polar lipid component refers to phosphatidylethanolamine (PE), phosphatidylserine (PS), phosphatidylcholine (PC), sphingomyelin (SM), and/or other phospholipids.

In the composition of the present invention, the phospholipid includes phosphatidylcholine, phosphatidylethanolamine and sphingomyelin, preferably including 0.5 to 1.5 parts by weight of phosphatidylethanolamine and 0.5 to 1.5 parts by weight of sphingomyelin with respect to 1 part by weight of phosphatidylcholine.

The composition may further include 1.35 wt% or less of a reducing sugar with respect to a dry weight of the composition. Preferably, 0.01 to 1.2 wt% of the reducing sugar with respect to the dry weight of the composition may be further included.

In the present invention, the reducing sugar refers to a sugar in which an aldehyde group or a ketone group of a sugar molecule exists in a free or hemiacetal form, and specifically, it may refer to a reducing sugar selected from glucose, fructose, sucrose, maltose and lactose.

In an embodiment, the whey-derived polar lipid concentrate of the present invention did not contain a reducing sugar. In another embodiment, it was confirmed that when lactose was added up to 1.2 wt% to the whey-derived polar lipid concentrate that does not contain such a reducing sugar, layer separation did not occur (see Table 2).

The composition of the present invention may further include 1 to 10 wt% of cholesterol with respect to the dry weight of the composition.

The composition of the present invention may further include 1 to 10 wt% of protein with respect to the dry weight of the composition.

As an example, the composition may further include 0.01 to 1.2 wt% of reducing sugar, 1 to 10 wt% of cholesterol, and 1 to 10 wt% of protein, with respect to the dry weight of the composition.

In the present invention, a spirit (e.g., alcohol, alcohol spirit, etc.) refers to an ethanol that may be drinkable by dilution, for example, starch raw material or sugar raw materials fermented and distilled or a distilled crude spirit.

The spirit includes less than 30 wt% of water, preferably in a range from 5 to 15 wt% of water, with respect to the total weight of the spirit. That is, an alcohol degree (concentration) of the spirit used in the present invention may be in a range from 85 to 95%. The alcohol degree (concentration) of the spirit is a volume concentration of ethanol. The alcohol degree of the spirit is a value obtained by dividing a volume of ethanol before mixing by a volume of an ethanol aqueous solution after mixing.

Another aspect of the present invention provides a method of preparing a composition of the present invention, the method including:
step 1) of preparing a whey protein concentrate by concentrating a whey solution;
step 2) of treating the whey protein concentrate with a water-containing spirit and then separating the whey protein concentrate into a spirit-soluble material and a spirit-insoluble material; and
step 3) of obtaining a whey-derived polar lipid-concentrated fraction by removing ethanol and moisture from the spirit-soluble material.

Step 1) may include preparing a crude whey protein solution by removing non-protein components such as lactose, minerals, vitamins, and inorganic components through a method such as performing ion exchange filtering, ultra-filtering, and micro filtering on the whey solution.

In an embodiment of the present invention, the protein content was concentrated to 90% or more by applying ion exchange filtering to the whey solution.

Step 1) may include preparing a whey protein concentrate by ultra-filtering the crude whey protein solution to a molecular weight of 10,000 (MWCO, Molecular weight cut off range) or less to separate it into a filtrate and a residue.

In step 2), the spirit treatment is preferably a step of mixing the whey protein concentrate with a spirit in an amount of 3 to 10 times a weight of the whey protein concentrate and extracting it at 5 to 80 °C, preferably 20 to 45 °C, for 0.5 to 12 hours.

In step 2), when the whey protein concentrate is treated with the water-containing spirit, it is separated into a spirit-soluble material and a spirit-insoluble material due to a difference in polarity.

In step 2), the spirit includes less than 30 wt% of water with respect to the total weight of the spirit, and preferably contains 5 to 15 wt% of water. That is, in the present invention, the alcohol degree of the spirit may be in a range from 85 to 95%.

The step 2) may include, after the spirit treating, holding it still at 30 to 50°C to remove neutral lipids.

The composition of the present invention may exist as a single-phase colloid in which phase separation does not occur regardless of temperature. The composition of the present invention is soluble in both hexane and toluene, which are non-polar organic solvents, and a content of insoluble material is less than 3 wt%.

In addition, the composition of the present invention may be used for emulsification and sensory enhancement purposes in food for infants and toddlers, food for special use, coffee drinks, and the like that retain the sensory characteristics of milk.

In an embodiment of the present invention, the composition may be a composition for improving intestinal health. Specifically, it may be a composition used for at least one use of inhibiting growth of harmful microorganisms in intestines, promoting growth of beneficial bacteria in the intestines, and alleviating inflammation of intestinal epithelial cells.

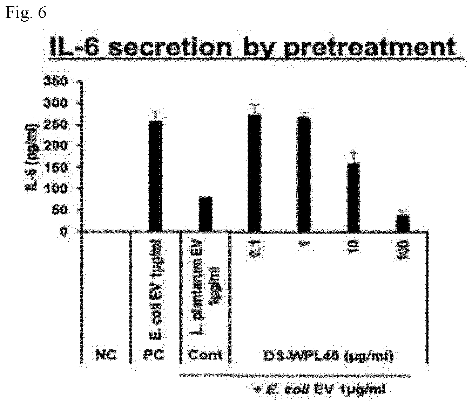

In a test example of the present invention, treating inflamed macrophages with the whey-derived polar lipid concentrate of the present invention resulted in a decrease of a secretion amount of a proinflammatory factor (e.g., inflammatory factor) IL-6, when the concentration was 10 and 100 µg/ml (Fig. 6).

In addition, in an embodiment of the present invention, the composition of the present invention may be a dry fraction having an iodine number of 10 or less by hydrogenation in a hydrogen pressurized environment using a palladium or nickel catalyst.

The fraction may be used for cosmetic purposes after undergoing a corresponding hydrogenation process.

In an embodiment of the present invention, the composition of the present invention may be used for antioxidant purposes as it is mixed in a range of 0.1 to 50 wt% in polyunsaturated fatty acids derived from fish oil, algae oil, and plant oil.

In addition, in an embodiment of the present invention, the composition of the present invention may exhibit physiological activity to promote myelinization, improve cognitive function, and improve sleep quality.

In addition, in an embodiment of the present invention, the composition of the present invention may be used in cosmetics for emulsification for micelles and liposomes.

In addition, in an embodiment of the present invention, the composition of the present invention may be used for emulsification and sensory enhancement in food for infants and toddlers, food for special purposes and coffee drinks.

In addition, the composition of the present invention may be a composition for food. The food may be selected from, for example, products such as cream, yogurt, drinking yogurt, cheese, cheese spreads, dairy products, soups, confectionery and bakery products.

In addition, the composition of the present invention may be a composition for cosmetics.

In addition, the composition of the present invention may be a pharmaceutical composition for improving intestinal health. The composition may be used in pharmaceuticals for improving intestinal health.

### MODES FOR IMPLEMENTATION OF THE INVENTION

Hereinafter, the present invention will be described in more detail by disclosing Embodiments and Test Examples. In Embodiments and Test Examples, "%" may mean "percent by weight (wt%)," unless specified otherwise.

### Embodiment 1

A whey solution obtained as a byproduct of cheese production was concentrated to a range from 6 to 7 % on a solid basis, and then it was ultra-filtered to a molecular weight of 10,000 kd to separate a filtrate and a residue. After concentrating again and spray-drying the separated residue to prepare a whey protein concentrate, the whey protein concentrate was mixed with a spirit (95 % alcohol degree) of 6 times a weight of the whey protein concentrate and extracted at 25°C for 1 hour, followed by removing components that are not soluble in the spirit (e.g., spirit-insolubles), and then removing ethanol and moisture from components that are soluble in the spirit (e.g., spirit-solubles), and accordingly, a whey-derived polar lipid concentrate was obtained.

The obtained whey-derived polar lipid concentrate was subjected to high performance liquid chromatography analysis using chloroform, methanol, and acetic acid in a gradient mobile phase, and it was confirmed that 5 wt% of cholesterol and 28 wt% of total phospholipid content (PC 9 wt%, PE 8 wt%, SM 9 wt%, and the like) were obtained.

In addition, Kjeldahl analysis was performed to confirm that a protein content was 5 wt%.

Specifically, a proteolytic agent and sulfuric acid were added to the obtained whey-derived polar lipid concentrate sample, and then the mixture was decomposed with a Foss Tecator Digestor Auto 20, Foss Tecator Scrubber at 420°C for 90 minutes. After decomposition, analysis was performed with a Foss Kjeltec 8400, Foss Kjeltec Sampler 8420.

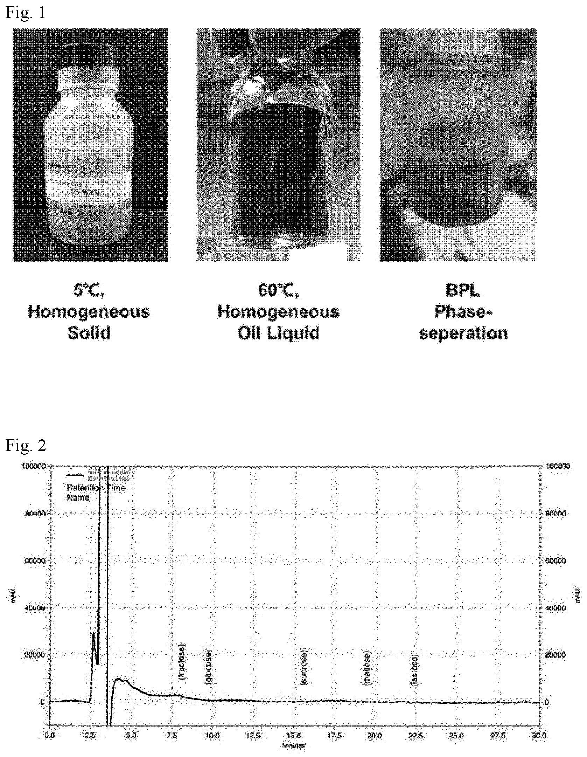

The obtained whey-derived polar lipid concentrate was subjected to high performance liquid chromatography analysis (flow rate: 1.0 ml/min, 35°C) for 30 minutes using acetonitrile (80 %) in a gradient mobile phase. Accordingly, it was confirmed that fructose, glucose, sucrose, maltose, lactose, and the like were not included (see FIG. 2).

In addition, ³¹P-NMR analysis was performed to reconfirm the phospholipid content.

### Comparative Example 1

A buttermilk powder containing lipids derived from a butter-oil preparing process was treated with a spirit having an alcohol degree of 95 % to be separated into a spirit-soluble material and a spirit-insoluble material, and then ethanol and moisture were removed from the spirit-soluble material to obtain a butter-derived polar lipid concentrate.

### Test Example 1

A crude fat content, a total lipid content, and a reducing sugar content such as lactose were evaluated in the whey-derived polar lipid concentrate of Embodiment 1 and the butter-derived polar lipid concentrate of Comparative Example 1, and the results are shown in Table 1 below.

**[Table 1]**

| Category | Whey-derived polar lipid-concentrate | Butter-derived polar lipid-concentrate |
|---|---|---|
| Crude fat content (Röse-Gottlieb method) | 99 wt% | 81 wt% |
| Total lipid content (Folch *et al,* solvent extraction) | 96 wt% | 73 wt% |
| Reducing sugar content | Not detected | 14 wt% |

As appreciated from the table above, the whey-derived polar lipid concentrate of the present invention had a total lipid content of 96 wt%, and no reducing sugar was detected.

### Test Example 2

Samples #1 to #10 including the whey-derived polar lipid concentrate of Embodiment 1 were added with different lactose contents as shown in Table 2 below, and the mixtures were heated and stirred at 50°C. Then, it was cooled to room temperature to determine whether it is in a single phase, and the results are shown in Table 2 below.

**[Table 2]**

| Category | #1 | #2 | #3 #4 | #5 | #6 | #7 | #8 | #9 | #10 |
|---|---|---|---|---|---|---|---|---|---|
| Lactose added (wt%) | 0.01 | 0.1 | 0.5 0.8 | 1 | 1.2 | 1.5 | 1.8 | 2 | 2.5 |
| Layer separation | None | None | None None | None | None | Occurred | Occurred | Occurred | Occurred |

Referring to the results of Table 2, it may be appreciated that layer separation does not occur when the composition contains lactose in an amount up to about 1.2 wt%.

### Embodiment 2

The whey-derived polar lipid concentrate of Embodiment 1 was treated with a 93% spirit containing water, and then left still at 30 to 50°C to further remove neutral lipids and some complex lipids (solid content: 15 wt %).

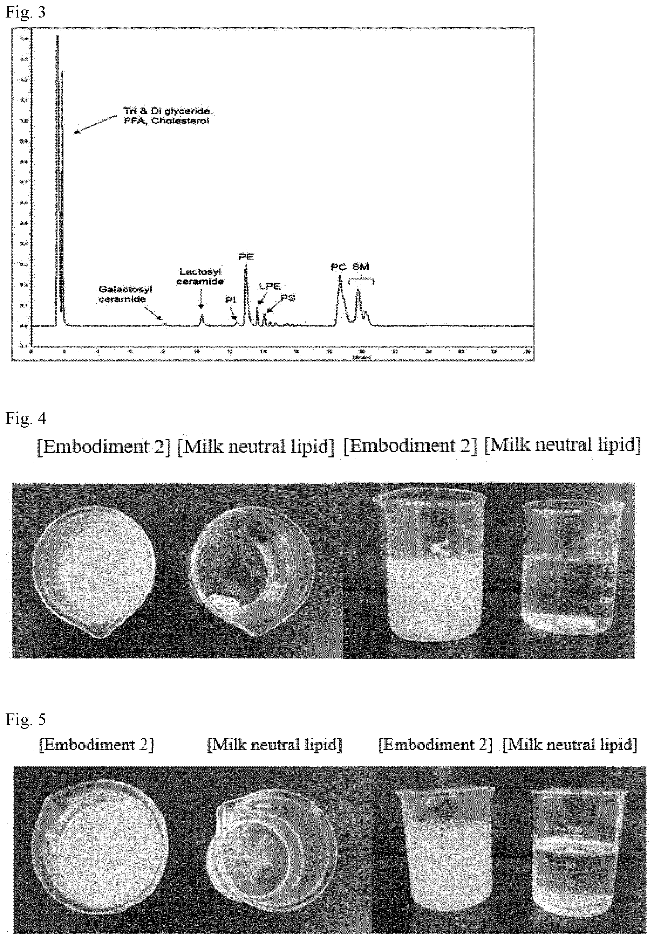

High performance liquid chromatography was performed to confirm that the total phospholipid content was 50 wt% (PC 18 wt%, PE 12 wt%, SM 18 wt%, and the like) (FIG. 3).

In addition, it was confirmed that the protein content was 5 wt% by performing Kjeldahl analysis, and it was confirmed that lactose and glucose were not included through carbohydrate analysis.

### Test Example 3: Change in polar lipid content according to extraction fraction

### characteristics

In the treating of the whey-derived polar lipid concentrate of Embodiment 1 with the water-containing spirit, a change in the content of polar lipids was measured, while varying an alcohol degree (%) and a solid content (wt %), and the results are shown in Table 3.

**[Table 3]**

| Category | Phospholipid | 95 % spirit | 94 % spirit | 93 % spirit | 90 % spirit | 85 % spirit |
|---|---|---|---|---|---|---|
| Solid content 8.5 wt% | PL (wt%) | 35 | 36 | 38 | 41 | 43 |
| | PC (wt%) | 10.8 | 11.5 | 12.4 | 13.3 | 14.3 |
| | PE (wt%) | 6.8 | 7.3 | 7.9 | 8.5 | 8.9 |
| | SM (wt%) | 9.8 | 10.4 | 11.3 | 12.1 | 12.7 |
| Solid content 12 wt% | PL (wt%) | 36 | 37 | 41 | 43 | 45 |
| | PC (wt%) | 10.9 | 11.9 | 12.6 | 13.7 | 14.4 |
| | PE (wt%) | 7.5 | 7.7 | 8.5 | 8.9 | 9.3 |
| | SM (wt%) | 10.7 | 11.0 | 12.1 | 12.7 | 13.3 |
| Solid content 15 wt% | PL (wt%) | 40 | 41 | 42 | 51 | 52 |
| | PC (wt%) | 12.1 | 12.5 | 13.1 | 17.1 | 19.5 |
| | PE (wt%) | 8.3 | 8.5 | 8.7 | 10.6 | 10.8 |
| | SM (wt%) | 11.9 | 12.1 | 12.4 | 15.1 | 15.4 |
| Solid content 16.6 wt% | PL (wt%) | 41 | 42 | 44 | 53 | 54 |
| | PC (wt%) | 13.3 | 14.2 | 15.6 | 17.7 | 20.5 |
| | PE (wt%) | 8.5 | 8.7 | 9.1 | 11.0 | 11.2 |
| | SM (wt%) | 12.1 | 12.4 | 13.0 | 15.7 | 16.0 |

In the table above, PL is a term collectively referring to phospholipids including PC, PE, SM, PS and PI, and PC/PE/SM are index phospholipids.

As shown in the table above, as a result of treating the polar lipid concentrate with the spirit, while changing the alcohol degree of the spirit to a range from 85 to 95 % and changing the solid content to a range from 8.5 to 16.6 wt%, the phospholipid content was at least 35 wt%.

As shown in the table above, the content of phospholipid, which is an index component of milk, changed according to the alcohol content (%) of the spirit and the solid content (wt%).

### Embodiment 3

The whey-derived polar lipid concentrate of Embodiment 2 was immersed in an acetone, dissolved by heating, and then cooled to be recrystallized. After separating the crystallized product and the acetone solution, only the crystallized product was separately obtained and analyzed by high performance liquid chromatography to confirm that it is a fraction having a total phospholipid content of 80 wt%.

### Test Example 4

The whey-derived polar lipid concentrate of Embodiment 2 and a milk neutral lipid were each dispersed in water and an MCT oil at a concentration of 1 % (v/v) at 40°C.

FIG. 4 illustrates a water-phase dispersion state of the whey-derived polar lipid-concentrated fraction of Embodiment 2 and the milk neutral lipid, and FIG. 5 illustrates an oil-phase dispersion state of the whey-derived polar lipid-concentrated fraction of Embodiment 2 and the milk neutral lipid.

As a result, it was confirmed that the whey-derived polar lipid concentrate of Embodiment 2 formed a milky white emulsion in the water and the MCT oil, and a phase separation occurred in the milk neutral lipid.

In addition, the whey-derived polar lipid concentrate of Embodiment 2 was dissolved in glycerin and water while heating at 80°C, then treated with a 1,000 rpm homomixer, and then subjected to 2,000 bar high-pressure emulsification twice, thereby obtaining a transparent emulsion. As a result of analyzing a zeta potential and a particle size of the emulsion using a Zetapal device, it was appreciated that the zeta potential value was -1.8 and the particle size was in a range from 100 to 150 nm.

### Embodiment 4

After dissolving the whey-derived polar lipid concentrate of Embodiment 2 with a spirit, a Pd/C catalyst in an amount of 10 % with respect to a percentage weight ratio of the concentrated fraction was added thereto, and then the mixture was stirred at 60°C and 30 bar under a hydrogen environment to add hydrogen.

The Pd/C catalyst was removed by filtration through a 0.2 µm membrane filter, cooled to 10°C, and then filtered with a Whatman No. 2 filter paper to separate a filtrate and a solid content. The solid content was concentrated under reduced pressure in a reduced-pressure concentrator to remove ethanol and moisture, and an iodine number analysis was then performed with a Wijs solution, and as a result, a fraction having an iodine number of 1 from which color and odor were removed was obtained.

### Test Example 5

The whey-derived polar lipid concentrate of Embodiment 2 was dispersed in a physiological saline, and inflammatory macrophages was treated therewith, thereby investigating the effect on the macrophages, that is, the effect of reducing secretion of the proinflammatory factor IL-6, and the effect of alleviating inflammation.

As a result, as illustrated in FIG. 6, it was appreciated that the secretion of the proinflammatory factor IL-6 was reduced when the concentration of the whey-derived polar lipid concentrate of Embodiment 2 was 10 and 100 µg/ml.

## Claims

1. A whey-derived polar lipid-concentrated fraction composition, being a single-phase composition derived from a whey protein concentrate,
wherein the composition is derived from a fraction obtained by treating the whey protein concentrate with a water-containing spirit,
a total lipid content is 90 percent by weight (wt%) or more with respect to a dry weight of the composition, and
a phospholipid content is 35 wt% or more with respect to the total lipid weight.

2. The composition of claim 1, further comprising a reducing sugar in an amount of 1.35 wt% or less with respect to the dry weight of the composition.

3. The composition of claim 2, wherein the reducing sugar is at least one sugar selected from the group consisting of glucose, fructose, sucrose, maltose and lactose.

4. The composition of claim 1, further comprising 1 to 10 wt% of cholesterol with respect to the dry weight of the composition.

5. The composition of claim 1, further comprising 1 to 10 wt% of protein with respect to the dry weight of the composition.

6. The composition of claim 1, wherein the phospholipid comprises phosphatidylcholine, phosphatidylethanolamine and sphingomyelin.

7. The composition of claim 6, wherein 0.5 to 1.5 parts by weight of the phosphatidylethanolamine and 0.5 to 1.5 parts by weight of the sphingomyelin are included with respect to 1 part by weight of the phosphatidylcholine.

8. The composition of claim 1, wherein the spirit comprises 5 to 15 wt% of water with respect to the total weight of the spirit.

9. The composition of any one of claims 1 to 8, wherein the composition is a composition for improving intestinal health.

10. The composition of claim 9, used for at least one use of inhibiting growth of harmful microorganisms in intestines, promoting growth of beneficial bacteria in the intestines, and alleviating inflammation of intestinal epithelial cells.

11. A method of preparing the whey-derived polar lipid-concentrated fraction composition according to claim 1, the method comprising:
1) preparing a whey protein concentrate by concentrating a whey solution;
2) treating the whey protein concentrate with a water-containing spirit and then separating the whey protein concentrate into a spirit-soluble material and a spirit-insoluble material; and
3) obtaining a whey-derived polar lipid-concentrated fraction by removing ethanol and moisture from the spirit-soluble material.

12. The method of claim 11, wherein in the treating with the spirit in step 2), the whey protein concentrate is mixed with the spirit which is in an amount of 3 to 10 times a weight of the whey protein concentrate and extracted at 5 to 80°C for 0.5 to 12 hours.

13. The method of claim 11, wherein the spirit comprises 5 to 15 wt% of water with respect to the total weight of the spirit.

14. The method of claim 11, further comprising, after the treating with the spirit in step 2), removing neutral lipids and part of complex lipids by holding the spirit-treated whey protein concentrate still at 30 to 50°C.

15. The method of claim 11, further comprising recrystallizing the whey-derived polar lipid-concentrated fraction in step 3).

16. The method of any one of claims 1 to 8, wherein the composition is a composition for food.

17. The method of any one of claims 1 to 8, wherein the composition is a composition for cosmetics.

18. The method of any one of claims 1 to 8, wherein the composition is a pharmaceutical composition for improving intestinal health.
